# DEMANDE DE BREVET EUROPEEN

(11) **EP 3 598 998 A1**
(43) Date de publication de la demande: **29.01.2020**
(21) Numéro de dépôt: 19188394.1
(22) Date de dépôt: 25.07.2019
(51) Int. Cl.: A61Q 3/02, A61K 8/37, A61K 8/55, A61K 8/73, A61K 8/87, A61K 8/891

(54) **COMPOSITIONS AMÉLIORÉES COMME PRODUIT DE FINITION APPLICABLE SUR LES ONGLES**

(30) Priorité: 27.07.2018 LU 100890
(71) Demandeur: International Lacquers S.A., 3225 Bettembourg (LU)
(72) Inventeur: VENTURA, Emmanuelle, 57000 Metz (FR); DEROSIER, Frédéric, 57680 Corny sur Moselle (FR); EDDOUMY, Fatima, 57840 Ottange (FR)
(74) Mandataire: Office Freylinger

(57) **Abrégé**

Composition de produit de finition pour les vernis à ongles comprenant au moins un solvant organique cosmétiquement acceptable, au moins un agent filmogène et un système de photoréticulation comprenant un ou plusieurs photoinitiateur(s) et au moins un composé réticulable (monomère, oligomère ou polymère) choisi parmi l'acryloyloxyéthyl butylcarbamate, le trimethylolpropane, un copolymère bis(pentaérythrityl triacrylate) pentaérythrityl diacrylate/IPDI (isophorone diisocyanate), le pentaérythrityl tétraacrylate, le pentaérythrityl triacrylate, un copolymère bis-HEMA (hydroxyéthyl méthacrylate) poly(1,4-butanediol)-9/IPDI, un copolymère bis-HEMA poly(1,4-butanediol)-9/IPDI, copolymère bis-HEMA polynéopentyl glycol adipate/IPDI, le di-HEMA triméthylhexyl dicarbamate, un copolymère HEMA/IPDI trimère isocyanurate/polycaprolactone diol et trimère tris-HEMA IPDI isocyanurate, un cross-polymère HEMA/IPDI trimère isocyanurate/PG, un copolymère hydroxyéthyl acrylate/IPDI/PPG-15 glycéryl éther, le PEG-4 trimethylolpropane triacrylate, le pentaérythrityl tétramercaptopropionate, l'uréthane acrylate, le 2-(phosphonooxy)éthyl méthacrylate, le bis(méthacryloyloxyethyl)-phosphate et acide phosphorique, le méthacrylate d'isobornyle, le triméthylolpropane triméthacrylate, le tripropylène glycol diacrylate et le PEG-4 diméthacrylate, et et un monomère et/ou oligomère (méth)acrylique.

## Description

### Domaine technique

La présente invention concerne de nouvelles compositions cosmétiques applicables sur les ongles en tant que produit de finition formant un film lisse, brillant et tenace avec une adhésion supérieure.

### Etat de la technique

Les compositions de finition ou top coat (selon la terminologie anglo-saxonne) s'appliquent sur le produit de maquillage des ongles, lui-même éventuellement appliqué sur un revêtement de base (ou base coat selon la terminologie anglo-saxonne). En effet, le produit de finition s'applique en général en dernière étape et peut être incolore ou coloré, avec différents effets de finition. A l'origine, le produit de finition est un vernis transparent et brillant que l'on applique sur le vernis coloré pour lui apporter brillance et résistance. De nos jours, le produit surpasse ces simples qualités de finition et de protection et propose également des effets décoratifs, notamment par exemple des glitters si un effet particulier est souhaité. Le procédé de maquillage des ongles, comprenant ces différentes étapes, incluant les temps de séchage, peut donc prendre beaucoup de temps.

Il existe dans le domaine des vernis à ongles en principe deux types de compositions qui se distinguent significativement par leur durée pendant laquelle elles peuvent rester sur l'ongle sans se détériorer significativement. Les vernis à ongles dits « conventionnels » (non réticulables) sont les bases dites « laque ou solvant » elles présentent souvent une adhésion de courte durée (typiquement 5 jours), mais on peut les retirer aisément. D'un autre côté, il existe les bases dites «gels UV» ou encore dites «soak-off» appliquées en institut spécialisé (uniquement professionnel) qui sont caractérisées par une très forte adhésion (3 semaines), mais peuvent induire des dommages importants de l'ongle (peeling) lors de leur retrait.

Les compositions dites « gels UV » sont des compositions liquides qui sous l'action d'un rayonnement lumineux provoquent des réactions de polymérisation et/ou de réticulation *in situ* aboutissant à des réseaux polymériques le plus souvent réticulés. De telles compositions photoréticulables sont communément appelées «gels UV».

Ce type de vernis à ongles est essentiellement constitué par des oligomères acryliques (de types acrylate, méthacrylate ou poly(éthylène glycol), de pigments, d'un photoinitiateur et de quantités faibles de solvant. Si la composition polymérique peut varier, leur modalité d'application reste la même. Sous l'effet d'un rayonnement UV, les photoinitiateurs amorcent un réaction de polymérisation des monomères et oligomères acryliques déposés sur l'ongle pour former un réseau polymérique complexe encapsulant les charges colorées et autres constituants particulaires. Il en résulte un film synthétique fin, compact et brillant capable de tenir plusieurs semaines sur l'ongle. L'intensité de réticulation est ajustée en fonction de la fonctionnalité du dépôt. Ainsi, la conception de faux ongles ou d'élongation de nature acrylique nécessite une densité de réticulation supérieure à un simple dépôt d'un vernis acrylique coloré. L'exceptionnelle adhésion et cohésion du film sur l'ongle sont dues d'une part à la densité du maillage polymérique formé combiné à une diffusion partielle des oligomères et monomères acryliques à la surface de l'ongle conduisant à une encapsulation partielle de la fibre de kératine présente à la surface de l'ongle. De ce fait, ce type de coating est généralement appliqué dans des instituts spécialisés à même de contrôler efficacement les méthodes de dépôt et le temps d'irradiation UV (« UV curing ») qui peuvent varier selon la nature des monomères et oligomères utilisés. Cette adhésion mécanique ou éventuellement chimique à la fibre de kératine peut induire un retrait du coating par peeling. Du fait de leur extrême stabilité, le retrait de ces bases acryliques ne se fait cependant pas sans dommage pour l'ongle. Les dommages induits par le retrait sont directement liés à la quantité de liens acryliques, entourant l'ongle et formés lors de la polymérisation.

Il est à noter qu'il existe sur le marché des gels UV pouvant être appliqués par les utilisateurs eux-mêmes. Pour favoriser leur tenue sur l'ongle, ils nécessitent cependant une étape de dépolissage de l'ongle afin de favoriser la tenue de la composition photoréticulée. De plus, ils nécessitent, lors du démaquillage, une étape de grattage de l'ongle par un outil métallique, une ponceuse électrique, ou une lime abrasive et/ou plonger les ongles pendant 5 à 10 minutes dans des solvants de type acétone. Ces étapes peuvent considérablement et durablement endommager l'ongle. Les gels UV nécessitent également une étape de dégraissage pour éliminer l'effet collant de la surface.

Les vernis à ongles dits «conventionnels» (non réticulables) sont les bases dites « laque ou solvant » qu'on peut aisément retirer, mais elles présentent souvent une adhésion de courte durée (typiquement 5 jours). D'un autre côté, les bases dites «gels UV» ou encore dites «soak-off» appliquées en institut spécialisé (uniquement professionnel) sont caractérisées par une très forte adhésion (3 semaines), mais peuvent induire des dommages importants de l'ongle (peeling) lors de leur retrait.

La tenue dans le temps des vernis à ongles constitue donc un enjeu majeur. Les deux paramètres critiques caractérisant cette tenue dans le temps sont l'adhésion du vernis à ongles et la brillance.

Le challenge majeur dans le domaine des compositions pour vernis à ongles reste donc le développement de formulations non toxiques, à tenue de longue durée, mais néanmoins détachables facilement.

En conséquence, il existe un besoin de développer des vernis à ongles longue durée ne présentant pas les inconvénients des «gels UV» déjà présents sur le marché, c'est-à-dire des vernis à ongles ne présentant pas de difficultés lors du retrait et n'induisant pas de dommages à l'ongle. Idéalement ces compositions permettent un maquillage de qualité dont la tenue et la brillance des compositions sont améliorées. En particulier, il existe une réelle demande de fournir des agents d'amélioration de la tenue et de la brillance de compositions de produit de maquillage des ongles compatibles avec tous les types de formulations de vernis à ongles, sans altérer (significativement) leurs autres propriétés. De préférence, ces compositions sont facilement démaquillables avec des démaquillants/dissolvants standards donc sans utilisation d'outils supplémentaires et sont, de plus, faciles à utiliser par les utilisateurs eux-mêmes.

### Objet de l'invention

Un objet de la présente invention est par conséquent de fournir une composition pour vernis à ongles en tant que produit de finition présentant une meilleure brillance et tenue de longue durée sur l'ongle tout en maintenant ses autres propriétés, à savoir : une grande facilité d'application, ainsi qu'un temps de séchage et de durcissement courts.

### Description générale de l'invention

Afin de résoudre le problème mentionné ci-dessus, la présente invention propose, dans un premier aspect, une composition de produit de finition pour les vernis à ongles comprenant au moins un solvant organique cosmétiquement acceptable, au moins un agent filmogène et un système de photoréticulation comprenant un ou plusieurs photoinitiateur(s) et au moins un composé réticulable (monomère, oligomère ou polymère) choisi parmi l'acryloyloxyéthyl butylcarbamate, le trimethylolpropane, un copolymère bis(pentaérythrityl triacrylate) pentaérythrityl diacrylate/IPDI (isophorone diisocyanate), le pentaérythrityl tétraacrylate, le pentaérythrityl triacrylate, un copolymère bis-HEMA (hydroxyéthyl méthacrylate) poly(1,4-butanediol)-9/IPDI, un copolymère bis-HEMA poly(1,4-butanediol)-9/IPDI, copolymère bis-HEMA polynéopentyl glycol adipate/IPDI, le di-HEMA triméthylhexyl dicarbamate, un copolymère HEMA/IPDI trimère isocyanurate/polycaprolactone diol et trimère tris-HEMA IPDI isocyanurate, un cross-polymère HEMA/IPDI trimère isocyanurate/PG, un copolymère hydroxyéthyl acrylate/IPDI/PPG-15 glycéryl éther, le PEG-4 trimethylolpropane triacrylate, le pentaérythrityl tétramercaptopropionate, l'uréthane acrylate, le 2-(phosphonooxy)éthyl méthacrylate, le bis(méthacryloyloxyethyl)phosphate et acide phosphorique, le méthacrylate d'isobornyle, le triméthylolpropane triméthacrylate, le tripropylène glycol diacrylate, le PEG-4 diméthacrylate et un monomère et/ou oligomère (méth)acrylique qui est choisi parmi les monomères suivants, respectivement parmi les oligomères comprenant de 2 à 10 unités monomériques dont au moins un des monomères suivants:
- les méthacrylates de formule CH₂ = C(CH₃)-COOR₁,
   dans laquelle R₁, représente un groupe alkyle non substitué, linéaire ou ramifié, contenant de 1 à 4 atomes de carbone, tel qu'un groupe méthyle, éthyle, propyle ou isobutyle ou R₁ représente un groupe cycloalkyle C₄ à C₁₂,
- les acrylates de formule CH₂ = CH-COOR₂
   dans laquelle R₂ représente un groupe cycloalkyle en C₄ à C₁₂ tel que l'acrylate d'isobornyle ou un groupe tertio butyle,
- les (méth)acrylamides de formule : 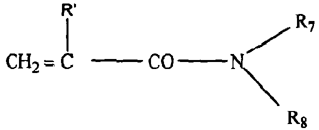 où R₇ et R₈ identiques ou différents représentent chacun un atome d'hydrogène ou un groupe alkyle de 1 à 12 atomes de carbone linéaire ou ramifié, tel qu'un groupe n-butyle, t-butyle, isopropyle, isohexyle, isooctyle, ou isononyle ; où R₇ représente H et R₈ représente un groupement 1,1-diméthyl-3-oxobutyl, et R' désigne H ou méthyle, et leurs mélanges.

Le au moins un composé réticulable comprend de préférence un copolymère IPDI/bis-HEMA polynéopentyl glycol adipate ou IPDI/bis-HEMA poly(1,4-butanediol)-9 ou leurs combinaisons.

Dans un deuxième aspect, l'invention propose également un kit (un nécessaire).

Dans un troisième aspect, l'invention propose un procédé de finition des ongles et/ou des faux-ongles.

Dans un quatrième aspect, l'invention concerne aussi l'utilisation d'une composition.

Les inventeurs ont découvert d'une manière surprenante que la combinaison d'une composition dite « conventionnelle » comprenant un agent filmogène, un solvant organique en association avec un système de photoréticulation comprenant des composés réticulables choisis parmi l'acryloyloxyéthyl butylcarbamate, le trimethylolpropane, un copolymère bis(pentaérythrityl triacrylate) pentaérythrityl diacrylate/IPDI (isophorone diisocyanate), le pentaérythrityl tétraacrylate, le pentaérythrityl triacrylate, un copolymère bis-HEMA (hydroxyéthyl méthacrylate) poly(1,4-butanediol)-9/IPDI, un copolymère bis-HEMA poly(1,4-butanediol)-9/IPDI, copolymère bis-HEMA polynéopentyl glycol adipate/IPDI, le di-HEMA triméthylhexyl dicarbamate, un copolymère HEMA/IPDI trimère isocyanurate/polycaprolactone diol et trimère tris-HEMA IPDI isocyanurate, un cross-polymère HEMA/IPDI trimère isocyanurate/PG, un copolymère hydroxyéthyl acrylate/IPDI/PPG-15 glycéryl éther, le PEG-4 trimethylolpropane triacrylate, le pentaérythrityl tétramercaptopropionate, l'uréthane acrylate, le 2-(phosphonooxy)éthyl méthacrylate, le bis(méthacryloyloxyethyl)phosphate et acide phosphorique, le méthacrylate d'isobornyle, le triméthylolpropane triméthacrylate, le tripropylène glycol diacrylate, le PEG-4 diméthacrylate et un monomère et/ou oligomère (méth)acrylique tel que défini ci-dessus, et un ou des photoinitiateurs, permettait d'améliorer très nettement la tenue en durée des compositions cosmétiques après application.

En effet, les vernis à ongles dits « conventionnels » ont comme avantage de s'appliquer et de se démaquiller très facilement et rapidement. Or, ces vernis à ongles s'écaillent très vite. D'un autre côté, les compositions gels UV réticulables présentent, elles, une tenue longue durée, mais restent difficiles à enlever avec comme risque de s'abimer les ongles. De plus, en raison de leurs résistances de longue durée, les utilisateurs ne peuvent modifier leur manucure à volonté.

La composition selon l'invention présente donc les avantages suivants:
- tenue de longue durée, bonne brillance,
- la composition est facilement et rapidement démaquillable avec des démaquillants standards,
- les utilisateurs peuvent, au gré de leur envie, modifier leur manucure aussi aisément qu'avec des vernis à ongles « conventionnels ».

Une composition n'est pas juste une addition d'ingrédients facilement remplaçable par d'autres et il est probable que l'ajout ou la substitution d'un composant affecte négativement d'autres propriétés de la formulation. Par conséquent, l'avantage de la présente invention consiste à proposer des familles d'ingrédient compatibles entre eux et qui s'influencent mutuellement pour obtenir un effet de tenue et de brillance sans compromettre l'intégrité de la formulation.

Un avantage additionnel important de la présente invention est que les compositions de produit de finition pour les ongles, de préférence les formulations de vernis à ongles, à tenue et brillance améliorées peuvent comprendre les ingrédients habituellement utilisés par l'homme de l'art. L'homme de métier n'est donc pas obligé de reconsidérer tous les ingrédients et leurs effets, s'il souhaite utiliser les présents ingrédients d'amélioration de la tenue et de la brillance. Les présents ingrédients d'amélioration de la tenue et de la brillance présentent par conséquent une très bonne compatibilité tant avec les solvants, qu'avec les ingrédients courants dans le domaine.

De façon préférée, la composition de produit de finition pour les ongles comprend un solvant organique. Ce solvant organique peut être choisi parmi les solvants usuels dans les vernis à ongles « conventionnels », de préférence dans le groupe constitué par le toluène, le xylène, l'acétate d'éthyle, l'acétate de butyle, l'acétate d'isobutyle, l'acétate de propyle, l'acétate de méthyle, une cétone, un ester, l'isopropanol, le butanol, le diacétone alcool, l'éthanol dénaturé (avec la méthyléthylcétone ou le diéthyléther), les hydrocarbures linéaires ou cycliques, comme par exemple l'hexane, le cyclohexane, l'heptane et n'importe quelle combinaison de ceux-ci.

Le solvant organique pourra être choisi en fonction de ses propriétés physico-chimiques et en considérations des autres composants présents dans la composition de produit de maquillage pour les ongles. De façon préférée, la quantité de solvant organique présente dans la composition de produit de finition pour les ongles est comprise entre 20 % et 80 % en poids par rapport au poids total de la composition.

Avantageusement, le solvant organique est l'acétate de butyle et/ou l'acétate d'éthyle.

La composition de produit de finition pour les ongles selon l'invention comprend également un agent filmogène. Le ou les agent(s) filmogène(s) pouvant être compris dans la composition de finition peu(ven)t être choisi(s) parmi le groupe constitué par la cellulose, un dérivé de cellulose, une résine vinylique, une résine acrylique et n'importe quelle combinaison de ceux-ci. Les dérivés de cellulose sont par exemple la nitrocellulose, l'acétate butyrate cellulose, l'éthylcellulose et l'hydroxy¬propyl¬cellulose. Préférentiellement, on choisira la nitrocellulose comme agent filmogène.

Un agent filmogène seul ou une combinaison d'agents filmogènes peut être utilisé(e) dans la composition de produit de maquillage pour les ongles dans les proportions allant de 2 % à 20 % en poids sec et de façon préférée de 5 % à 12 % en poids sec par rapport au poids total de la composition.

La composition de produit de finition pour les ongles comprend au moins un composé réticulable comme défini ci-dessus. La quantité de ce ou ces composés réticulables est comprise de 4 % à 35 % en poids sec par rapport au poids total de la composition.

La composition de produit de finition pour les ongles comprend également au moins un photoinitiateur qui est choisi parmi le groupe constitué des α-hydroxycétones, des α-aminocétones, des cétones aromatiques de préférences associées à un composé donneur d'hydrogène, des α-dicétones aromatiques et des oxydes d'acylphosphine, et de leurs mélanges, avantageusement dans le groupe constitué des oxydes d'acylphosphine.

La quantité du au moins un photoinitiateur est comprise de 0,2% à 10% en poids sec par rapport au poids total de la composition.

La composition peut comprendre une résine principale choisie parmi une résine polyester, une résine époxytosylamide et une résine acrylate et styrène-acrylate, les sucroses benzoate et perbenzoate et leurs combinaisons.

Des résines supplémentaires (différentes) peuvent également être ajoutées dans la composition. En particulier, ces résines supplémentaires permettent d'augmenter le collant et l'adhérence du film sur l'ongle.

Les résines pouvant être utilisées sont les suivantes :
- les résines tosylamide/formaldéhyde ou résines toluènesulfonamide/ formaldéhyde,
- les résines tosylamide/époxy ou résines toluènesulfonamide/époxy,
- les copolymères de glycérine/acide phtalique,
- les copolymères styrène/acrylate/acrylonitrile,
- les polymères et copolymères d'acrylates,
- les copolymères d'acrylates et de styrène,
- le sucrose acétate isobutyrate,
- les résines polyvinylbutyral.

La composition de produit de finition pour les ongles peut aussi comprendre au moins un plastifiant. Les plastifiants sont des composés ayant une température d'ébullition élevée qui ne s'évaporent pas lors du séchage du vernis à ongles. Ils sont utilisés pour moduler la dureté du film formé afin d'obtenir les caractéristiques physico-chimiques du film souhaitées et représentent en général de 0,1 % à 12 % en poids sec, de préférence 4 % à 8 % en poids sec par rapport au poids total de la composition. Le plastifiant est de préférence choisi parmi l'acétyl tributyl citrate, l'acétyl triéthyl citrate, le tributyl citrate, le triéthylcitrate, le dibutyl phtalate, le triphénylphosphate, la triacétine, le triméthyl pentanyl diisobutyrate, la triéthylhexanoïne, le sucrose benzoate, le dibutyl adipate, le diéthyl phtalate, le diisobutyl adipate, le diisopropyl adipate, le dipropylène glycol dibenzoate, le N-éthyl toluène sulfonamide, ses isomères ortho et para, le N-(2-hydroxypropyl) benzène sulfonamide et le N-(n-butyl) benzène sulfonamide, etc.

Pour fournir des effets particuliers à la composition en tant que produit de finition pour les ongles, elle peut comprendre un ou plusieurs agents de coloration choisis parmi les glitters (paillettes) et/ou les particules métalliques. Ces agents de coloration fournissent l'aspect esthétique recherché par les utilisateurs de vernis à ongles. Ces agents de coloration peuvent produire des effets très différents tels que « métallisé », « pailleté », « mat », « néon », « fluorescent », « holographique », etc.

Les glitters (paillettes) utilisables dans l'invention peuvent être préparés à partir de plusieurs types de matériaux comme les films polyesters comportant en surface une couche métallique, le polyéthylène téréphtalate, le polybutylène téréphtalate, les copolymères d'acrylates, les copolymères d'acrylates ou encore l'aluminium.

Selon un mode de réalisation de l'invention, la composition de produit de finition pour les ongles comprend une quantité d'agent de coloration comprise de 0,1 % à 20 % en poids sec et de préférence de 0,5 % à 14 % en poids sec par rapport au poids total de la composition.

La composition en tant que produit de finition pour les ongles peut en outre comprendre divers additifs choisis parmi les modificateurs de surface et les agents dits « traitants ».

Parmi les additifs utilisables selon l'invention, on citera les modificateurs de surface comme les cyclométhicones ou cyclopentasiloxane, les diméthicones et le triméthylsiloxysilicate.

Ces additifs de surface sont généralement utilisés dans des faibles quantités dans la formulation allant de 0,05 à 5 % en poids sec par rapport au poids total de la composition.

On citera également les additifs dits « traitants » de l'ongle utilisables dans la composition selon l'invention. Les additifs traitants peuvent représenter de 0,01 % à 5 % en poids sec, de préférence de 0,05 % à 2 % en poids sec par rapport au poids total de la composition. Parmi ces additifs « traitants », on citera:
- le formaldéhyde utilisé comme durcisseur de l'ongle,
- la vitamine E acétate (tocophéryl acétate),
- la vitamine A palmitate (rétinyl palmitate),
- les dérivés organiques ou inorganiques de calcium tels que le chlorure de calcium, le pantothénate de calcium,
- le diméthyloxobenzodioxasilane,
- la myrrhe,
- les acides aminés soufrés comme la cystéine, ses sels et leurs dérivés, la cystine, le glutathion. Les acides aminés soufrés sont en effet capables de créer des ponts de réticulation entre la kératine et les groupes SH libres de ces dérivés soufrés,
- la kératine hydrolysée d'origine végétale,
- les alpha-hydroxyacides comme l'acide citrique, l'acide ascorbique,
- la biotine,
- l'urée et la diméthylurée.

Un autre objet de l'invention concerne un produit de finition pour les ongles comprenant une composition telle que décrite précédemment.

La présente invention concerne un kit comprenant au moins un revêtement de base, un produit de maquillage pour les ongles selon l'invention et un produit de finition.

Un kit au sens de la présente invention signifie par exemple que les première, deuxième compositions et (éventuellement) la troisième composition sont commercialisées sous un même conditionnement, ou dans deux ou (éventuellement) trois conditionnements séparées sous un même emballage, ou dans deux ou (éventuellement) trois conditionnements séparés sous leur emballage respectif avec une indication d'utilisation combinée de la première composition et de la deuxième composition et (éventuellement) la troisième.

Selon un mode de réalisation particulier, les éléments du kit selon l'invention sont destinés à être appliqués sur les ongles et/ou faux-ongles selon le procédé de l'invention.

Selon un troisième aspect, l'invention propose un procédé de finition des ongles et/ou des faux-ongles comprenant au moins les étapes suivantes:
a) optionnellement, application sur un ongle ou un faux-ongle d'une première composition d'un kit conforme à l'invention, par laquelle on dépose un premier revêtement de base constitué d'au moins une couche d'une composition photoréticulable ou non, ce premier revêtement de base étant appliqué directement au contact de l'ongle ou du faux-ongle, et
b) optionnellement si le premier revêtement comprend une composition photoréticulable, exposition de l'ongle ou du faux-ongle revêtu obtenu à l'issue de l'étape a) à un rayonnement lumineux, UV ou visible,
c) application sur l'éventuel premier revêtement d'une composition de maquillage des ongles d'un kit conforme à l'invention, par laquelle on dépose un deuxième revêtement constitué d'au moins une couche de ladite deuxième composition, de préférence un produit de maquillage photoréticulable ou non,,
d) optionnellement si le deuxième revêtement comprend une composition photoréticulable, exposition de l'ongle ou du faux-ongle revêtu obtenu à l'issu de l'étape c) à un rayonnement lumineux, UV ou visible,
e) application sur le deuxième revêtement issu des étapes c) à d), d'un troisième revêtement constitué d'au moins une couche d'une troisième composition de produit de finition selon l'invention,
f) exposition de l'ongle ou du faux-ongle revêtu obtenu à l'issu de l'étape e) à un rayonnement lumineux, UV ou visible.

Selon un mode de réalisation, le procédé de l'invention comprend en outre, avant l'étape b), une période de séchage du revêtement déposé à l'issue de l'étape a), dont la durée peut varier de 10 secondes à 15 minutes, typiquement de 5 minutes à 10 minutes. Ledit séchage est généralement effectue à l'air et à la température ambiante. Puis à l'étape b) 10 secondes à 5 minutes sous le rayonnement lumineux, UV ou visible

Le revêtement réticulé issu de la réticulation de l'étape f) présente une tenue dans le temps, en termes de résistance à l'écaillement et de brillance, significative et notamment à l'échelle d'au moins 7 à 20 jours. Il s'avère ainsi résistant à l'usure ni d'écaillage significatifs dans ce délai.

Ces revêtements possèdent également une aptitude à se solubiliser ou à augmenter de volume, lorsqu'il est mis en contact avec un solvant de démaquillage usuel. Cette aptitude à se solubiliser ou à gonfler, manifestée par le revêtement réticulé, est précisément avantageuse pour son élimination lorsque celui-ci est appliqué en surface d'un ongle ou d'un faux-ongle. En effet, les revêtements peuvent être aisément éliminés par simple démaquillage à l'aide d'un dissolvant classique.

Un quatrième aspect de l'invention concerne l'utilisation de résine choisie parmi une résine de et leurs combinaisons en association avec un système de photoréticulation comprenant un ou des composés réticulables tels que décrits ci-dessus et un ou des photoinitiateurs, pour améliorer la tenue en durée des compositions de produit de finition après application en combinaison avec un démaquillage au dissolvant.

### Exemple

La composition en tant que produit de finition suivante a été préparée:

| Ingrédient | Quantité (% en poids) |
|---|---|
| acétate d'éthyle | 29,10 |
| acétate de butyle | 22,50 |
| Alcool dénaturé | 15,00 |
| nitrocellulose | 11,20 |
| alcool isopropylique | 4,80 |
| copolymères d'IPDI/bis-HEMA polynéopentyl glycol adipate | 3,75 |
| copolymères d'IPDI/bis-HEMA poly(1,4-butanediol)-9 | 3,00 |
| (méth)acrylate d'isobornyle | 5,30 |
| Dipropylène glycol dibenzoate | 3,00 |
| triméthylpropane triméthacrylate | 1,25 |
| Ethyl triméthylbenzoyl phenylphosphinate | 1,00 |
| diméthicone | 0,10 |

La composition en tant que produit de finition pour les ongles, selon l'invention, a été préparée.

Il y a plus d'apport d'adhésion au vernis à ongles par rapport à la composition de revêtement classique. On observe une résistance au solvant qui est bien meilleure.

On se fixe une dureté plus basse, mesurée après 12 h à 32 °C, 150 environ contre 200-300 pour un vernis à ongles classique. Les brillances sont, par contre, comparables mais la brillance est maintenue dans le temps par rapport à un vernis classique, on améliore ici la tenue de la formule.

Par rapport à une composition de revêtement classique pour lequel l'adhésion quadrillage est bonne mais tout le film part après application du scotch, ici l'adhésion au scotch est bien améliorée, le quadrillage s'abîme légèrement mais rien de comparable à composition de revêtement classique.

## Revendications

1. Composition de produit de finition pour les vernis à ongles comprenant au moins un solvant organique cosmétiquement acceptable, au moins un agent filmogène et un système de photoréticulation comprenant un ou plusieurs photoinitiateur(s) et au moins un composé réticulable choisi parmi l'acryloyloxyéthyl butylcarbamate, le trimethylolpropane, un copolymère bis(pentaérythrityl triacrylate) pentaérythrityl diacrylate/IPDI (isophorone diisocyanate), le pentaérythrityl tétraacrylate, le pentaérythrityl triacrylate, un copolymère bis-HEMA (hydroxyéthyl méthacrylate) poly(1,4-butanediol)-9/IPDI, un copolymère bis-HEMA poly(1,4-butanediol)-9/IPDI, copolymère bis-HEMA polynéopentyl glycol adipate/IPDI, le di-HEMA triméthylhexyl dicarbamate, un copolymère HEMA/IPDI trimère isocyanurate/polycaprolactone diol et trimère tris-HEMA IPDI isocyanurate, un cross-polymère HEMA/IPDI trimère isocyanurate/PG, un copolymère hydroxyéthyl acrylate/IPDI/PPG-15 glycéryl éther, le PEG-4 trimethylolpropane triacrylate, le pentaérythrityl tétramercaptopropionate, l'uréthane acrylate, le 2-(phosphonooxy)éthyl méthacrylate, le bis(méthacryloyloxyethyl)phosphate et acide phosphorique, le méthacrylate d'isobornyle, le triméthylolpropane triméthacrylate, le tripropylène glycol diacrylate, le PEG-4 diméthacrylate, et un monomère et/ou oligomère (méth)acrylique qui est choisi parmi les monomères suivants, respectivement parmi les oligomères comprenant de 2 à 10 unités monomériques dont au moins un des monomères suivants:
- les méthacrylates de formule CH₂ = C(CH₃)-COOR₁,
dans laquelle R₁, représente un groupe alkyle non substitué, linéaire ou ramifié, contenant de 1 à 4 atomes de carbone, tel qu'un groupe méthyle, éthyle, propyle ou isobutyle ou R₁ représente un groupe cycloalkyle C₄ à C₁₂,
- les acrylates de formule CH₂ = CH-COOR₂
dans laquelle R₂ représente un groupe cycloalkyle en C₄ à C₁₂ tel que l'acrylate d'isobornyle ou un groupe tertio butyle,
- les (méth)acrylamides de formule : 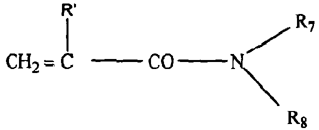 où R₇ et R₈ identiques ou différents représentent chacun un atome d'hydrogène ou un groupe alkyle de 1 à 12 atomes de carbone linéaire ou ramifié, tel qu'un groupe n-butyle, t-butyle, isopropyle, isohexyle, isooctyle, ou isononyle ; où R₇ représente H et R₈ représente un groupement 1,1-diméthyl-3-oxobutyl, et R' désigne H ou méthyle, et leurs mélanges.

2. Composition de produit de finition pour les ongles selon la revendication 1, **caractérisée en ce que** le au moins un composé réticulable est choisi parmi le copolymère IPDI/bis-HEMA polynéopentyl glycol adipate ou IPDI/bis-HEMA poly(1,4-butanediol)-9 et leurs combinaisons.

3. Composition de produit de finition pour les ongles selon l'une quelconque des revendications précédentes, **caractérisée en ce que** la quantité de composé réticulable est comprise entre 4 % et 12 % en poids sec par rapport au poids total de la composition.

4. Composition de produit de finition pour les ongles selon l'une quelconque des revendications précédentes, comprenant au moins un solvant organique choisi parmi le toluène, le xylène, l'acétate d'éthyle, l'acétate de butyle, l'acétate d'isobutyle, l'acétate de propyle, l'acétate de méthyle une cétone, un ester, l'isopropanol, le butanol le diacétone alcool, l'éthanol dénaturé avec la méthyléthylcétone ou le diéthyléther, les hydrocarbures linéaires ou cycliques, de préférence le cyclohexane, l'heptane ou n'importe quelle combinaison de ceux-ci, de préférence le solvant organique est l'acétate d'éthyle, l'acétate de butyle ou une combinaison de ceux-ci, la quantité de solvant organique étant de préférence comprise entre 20 % et 80 % en poids par rapport au poids total de la composition.

5. Composition de produit de finition pour les ongles selon l'une quelconque des revendications précédentes, **caractérisée en ce que** le au moins un photoinitiateur est choisi parmi le groupe constitué des α-hydroxycétones, des α-aminocétones, des cétones aromatiques de préférences associées à un composé donneur d'hydrogène, des α-dicétones aromatiques et des oxydes d'acylphosphine, et de leurs mélanges, avantageusement dans le groupe constitué des oxydes d'acylphosphine, la quantité du photoinitiateur étant de préférence comprise de 0,2% à 10% en poids sec par rapport au poids total de la composition.

6. Composition de produit de finition pour les ongles selon l'une quelconque des revendications précédentes, **caractérisée en ce que** l'agent filmogène est choisi parmi la cellulose, un dérivé de cellulose, une résine vinylique, ou n'importe quelle combinaison de ceux-ci, de préférence l'agent filmogène est la nitrocellulose.

7. Composition de produit de finition pour les ongles selon l'une quelconque des revendications précédentes, **caractérisée en ce que** la quantité d'agent filmogène est comprise de 2 % à 20 % en poids sec et de façon préférée de 5 % à 12 % en poids sec par rapport au poids total de la composition.

8. Composition de produit de finition pour les vernis à ongles selon l'une quelconque des revendications précédentes, comprenant en outre une résine principale choisie parmi une résine polyester, une résine époxytosylamide et une résine acrylate et styrène-acrylate, les sucroses benzoate et perbenzoate et leurs combinaisons.

9. Composition de produit de finition pour les vernis à ongles selon la revendication 10, comprenant en outre une ou plusieurs résines auxiliaires différente de la ou des résines principales, choisies parmi les résines tosylamide/formaldéhyde ou résines toluènesulfonamide/ formaldéhyde, les résines tosylamide/époxy ou résines toluènesulfonamide/époxy, la résine de copolymères d'acide adipique, d'acide fumarique, de néopentylglycol et de cyclohexanediméthanol, les copolymères acide adipique/néopentyl glycol/anhydride trimellitique, les copolymères d'anhydride phtalique/ glycérine/glycidyl décanoate, les copolymères d'anhydride phtalique/ anhydride trimellitique/glycols, les copolymères de glycérine/acide phtalique, les copolymères styrène/acrylate/acrylonitrile, les polymères et copolymères d'acrylates, les copolymères d'acrylates et de styrène, la sucrose acétate isobutyrate, la résine polyvinylbutyral.

10. Composition de produit de finition pour les ongles selon l'une quelconque des revendications précédentes, comprenant en outre un ou plusieurs additifs choisis parmi les résines, les plastifiants, les modificateurs de surface et les agents dits « traitants ».

11. Composition de produit de finition pour les ongles selon l'une quelconque des revendications précédentes, **caractérisée en ce qu'**elle comprend en outre un ou plusieurs agents de coloration choisis parmi les glitters et/ou les particules métalliques.

12. Produit de finition pour les ongles comprenant une composition selon l'une quelconque des revendications précédentes.

13. Kit comprenant une composition de produit de finition pour les ongles selon l'une quelconque des revendications 1 à 11 ou un produit de finition pour les ongles selon la revendication 12, et comprenant éventuellement en outre un revêtement de base et un produit de maquillage.

14. Procédé de finition des ongles et/ou des faux-ongles facilement démaquillable comprenant les étapes suivantes:
(a) optionnellement, application sur un ongle ou un faux-ongle d'une première composition de revêtement de base d'un kit selon la revendication 13, par laquelle on dépose un premier revêtement de base constitué d'au moins une couche d'une composition photoréticulable ou non, ce premier revêtement de base étant appliqué directement au contact de l'ongle ou du faux-ongle, et
(b) optionnellement si le premier revêtement comprend une composition photoréticulable, exposition de l'ongle ou du faux-ongle revêtu obtenu à l'issue de l'étape a) à un rayonnement lumineux, UV ou visible,
(c) application sur l'éventuel premier revêtement d'une composition de maquillage des ongles d'un kit selon la revendication 13, par laquelle on dépose un deuxième revêtement de produit de maquillage pour les ongles,
(d) optionnellement si le deuxième revêtement comprend une composition photoréticulable, exposition de l'ongle ou du faux-ongle revêtu obtenu à l'issu de l'étape c) à un rayonnement lumineux, UV ou visible,
(e) application sur le deuxième revêtement issu des étapes c) à d), d'un troisième revêtement constitué d'au moins une couche d'une troisième composition de produit de finition photoréticulable selon l'une quelconque des revendications 1 à 11,
(f) exposition de l'ongle ou du faux-ongle revêtu obtenu à l'issu de l'étape e) à un rayonnement lumineux, UV ou visible.

15. Utilisation d'au moins un composé réticulable choisi parmi l'acryloyloxyéthyl butylcarbamate, le trimethylolpropane, un copolymère bis(pentaérythrityl triacrylate) pentaérythrityl diacrylate/IPDI (isophorone diisocyanate), le pentaérythrityl tétraacrylate, le pentaérythrityl triacrylate, un copolymère bis-HEMA (hydroxyéthyl méthacrylate) poly(1,4-butanediol)-9/IPDI, un copolymère bis-HEMA poly(1,4-butanediol)-9/IPDI, copolymère bis-HEMA polynéopentyl glycol adipate/IPDI, le di-HEMA triméthylhexyl dicarbamate, un copolymère HEMA/IPDI trimère isocyanurate/polycaprolactone diol et trimère tris-HEMA IPDI isocyanurate, un cross-polymère HEMA/IPDI trimère isocyanurate/PG, un copolymère hydroxyéthyl acrylate/IPDI/PPG-15 glycéryl éther, le PEG-4 trimethylolpropane triacrylate, le pentaérythrityl tétramercaptopropionate, l'uréthane acrylate, le 2-(phosphonooxy)éthyl méthacrylate, le bis(méthacryloyloxyethyl)phosphate et acide phosphorique, le méthacrylate d'isobornyle, le triméthylolpropane triméthacrylate, le tripropylène glycol diacrylate et le PEG-4 diméthacrylate, pour améliorer la tenue en durée de compositions ou de produits de finition pour les ongles après application en combinaison avec un démaquillage au dissolvant.
